# EUROPEAN PATENT SPECIFICATION

(11) **EP 3 356 337 B1**
(45) Date of publication and mention of the grant of the patent: **24.06.2020**
(21) Application number: 16777751.5
(22) Date of filing: 28.09.2016
(51) Int. Cl.: A61K 31/554, A61P 25/08

(54) **TRICYCLIC DIBENZOTHIAZEPINE TYPE COMPOUNDS FOR USE IN THE THERAPY OF CDKL5 DISORDER**
TRICYCLISCHE DIBENZOTHIAZEPINVERBINDUNGEN ZUR ANWENDUNG IN DER THERAPIE VON CDKL5-STÖRUNGEN
UTILISATION DE COMPOSÉS DE TYPE DIBENZOTHIAZÉPINE TRICYCLIQUE POUR LE TRAITEMENT DU SYNDROME DU CDKL5

(30) Priority: 28.09.2015 GB 201517133
(43) Date of publication of application: 08.08.2018
(73) Proprietor: Healx Limited, Cambridge, Cambridgeshire CB3 0DU (GB)
(72) Inventor: CAVALLA, David, Cambridge Cambridgeshire CB4 0WS (GB)
(74) Representative: Potter Clarkson
(86) International application number: PCT/GB2016/053007
(87) International publication number: WO 2017/055834

(56) References cited:
- WO-A1-2012/143703
- B S MCEWEN ET AL: "The neurobiological properties of tianeptine (Stablon): from monoamine hypothesis to glutamatergic modulation", MOLECULAR PSYCHIATRY, vol. 15, no. 3, 25 August 2009 (2009-08-25), pages 237-249, XP055312956, GB ISSN: 1359-4184, DOI: 10.1038/mp.2009.80
- CLAUDIA FUCHS ET AL: "Inhibition of GSK3? rescues hippocampal development and learning in a mouse model of CDKL5 disorder", NEUROBIOLOGY OF DISEASE, vol. 82, 2 July 2015 (2015-07-02), pages 298-310, XP055386961, AMSTERDAM, NL ISSN: 0969-9961, DOI: 10.1016/j.nbd.2015.06.018

## Description

### Field of the invention

The present invention relates to tricyclic dibenzothiazepine type compounds for use in the treatment of CDKL5 disorder in a subject (i.e. a mammal such as an animal or human, especially a human) suffering from such disorder. In particular, although not exclusively, the present invention relates to alleviating CDKL5 disorder with tricyclic dibenzothiazepine type compounds of Formula I in a subject as a result of a medical condition. Additionally, the present invention relates to pharmaceutical compositions including tricyclic dibenzothiazepine type compounds of Formula I and the use of such compositions as a medicament, particularly a human medicament.

### Background of the invention

Compounds of Formula I where X=CH₂ include tianeptine, which is originally described in French patent 2,104,728 and has been reported that it may be used in the treatment of neurodegenerative pathologies, neuropathic pain, fibromyalgia, chronic fatigue syndrome and irritable bowel syndrome. Similarly compounds of Formula I where X=O or S are described in WO2012143703, which is incorporated herein by reference. The effects of compounds of Formula I on CDKL5 disorder have not been previously described.

Cyclin-dependent kinase-like 5 CDKL5 is a serine/threonine (S/T) kinase that is highly expressed in the brain. Mutations in the X-linked CDKL5 gene cause early-onset epileptic encephalopathy. Although CDKL5 disorder shares several features with Rett Syndrome, a neurodevelopmental disorder caused by mutations in the X-linked MECP2 gene, recent work assessing data has argued that it should be considered a distinct clinical entity, primarily due to its early onset and lack of clinical regression following a period of normal development (Fehr S, et al. (2013) Eur J Hum Genet 21 (3) 266-273). The two conditions are also characterized by different genotypes, with Rett's syndrome being associated with a defect in *Mecp2* while CDKL5 disorder involves defects in *Cdkl5* gene. The primary clinical features of CDKL5 disorder are early life seizures, motor rigidity, dyskinesias, stereotypical hand movements, and deficient language acquisition. Additional phenotypical characteristics include impaired learning ability, impaired memory, respiratory dysfunction and aspects of autistic behaviour. Several additional features have been noted in some carriers, including gastrointestinal problems, bruxism, and a characteristic sideways glance (Bahi-Buisson N et al. (2008) Brain 131: 2647-2661). The disorder is most frequently associated with nonsense or putative detrimental missense mutations and is thought to be caused by a loss of CDKL5 function, although no clear relationship between the type or location of mutations and symptom severity has been reported. The disorder is more frequently reported in females (8:1), probably due to the more severe consequences of dominant X-linked mutations in males than in females. Recent evidence suggests that CDKL5 is involved in neuronal plasticity. The modulation of CDKL5 alongside neuronal maturation has been measured using markers of pre- and post-synaptic development, Synapsin 1 and GluR2 (see Montanara et al, J Biol Chem. 2015; 290(7): 4512-4527, the contents of which are incorporated by reference). GluR2 is a subunit of the AMPA receptor which influences receptor assembly and trafficking and plays a pivotal role in long-term synaptic plasticity. As *GluR2,* it is one of the CREB (cAMP response element-binding protein)-responsive immediate early genes. GluR2 is also a post-synaptic marker.

### Summary of the invention

According to a first aspect, the present invention provides a compound of Formula I, or a pharmaceutically or veterinarily acceptable salt thereof, or a pharmaceutically or veterinarily acceptable solvate of either entity or a pharmaceutical or veterinary composition containing any of the foregoing for use in the treatment of a motor deficit in a subject with CDKL5 disorder (i.e. mammal such as an animal or human, especially a human), wherein a compound of Formula I comprises: 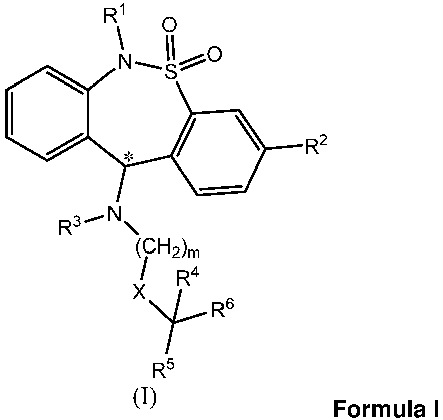 or a pharmaceutically or veterinarily acceptable salt thereof, or a pharmaceutically or veterinarily acceptable solvate of either entity,
wherein:
R¹ and R³ each independently represent, at each occurrence when used herein,
H or C₁ to C₅ alkyl;
R² represents halo;
R⁴ and R⁵ each independently represent H;
R⁶ represents -C(O)OR⁹;
X represents CH₂, O or S;
R⁹ represents H or C₁ to C₅ alkyl;
and,
m is an integer from 1 to 6 inclusive.

### Description of the Figures

Figure 1 is a Western blot showing how administration of tianeptine sulphate affects CDKL5 silencing and its subsequent effect on expression of the AMPA receptor subunit GluR2 and on phosphorylated GluR2 (phosphorylation at serine-880).
Figure 2 shows the results of an immunofluorescence study investigating how administration of tianeptine sulphate affects CDKL5 silencing and its subsequent effect on expression of the AMPA receptor subunit GluR2.
Figure 3 is Western Blot showing how administration of 2-(4-((3-chloro-6-methyl-5,5-dioxido-6,11-dihydrodibenzo[c,f][1,2]thiazepin-11-yl)amino)butoxy)acetic acid, HCI salt (TIOX) affects CDKL5 silencing and its subsequent effect on expression of the AMPA receptor subunit GluR2 and on phosphorylated GluR2 (phosphorylation at serine-880).

### Description of Preferred Embodiments

By the term "treatment" or "treating" as used herein, we include both therapeutic (curative), palliative and prophylactic treatment. In other words, as used herein, the term treatment includes "prevention". Suitably, the treatment of the CDKL5 disorder is accomplished by administration of a therapeutically effective amount of a compound of Formula I, or a pharmacologically active metabolite thereof, or a pharmaceutically or veterinarily acceptable salt thereof, or a pharmaceutically or veterinarily acceptable solvate of either entity or a pharmaceutical or veterinary composition containing any of the foregoing to the subject.

The term "effective amount" or "therapeutically effective amount" as used herein refers to the amount or dosage of an agent sufficient to effectuate a desired therapeutic effect. Such amount may vary depending on the effect to be achieved, the agent used and the body weight of the subject. Typically, a therapeutically effective amount of a compound of Formula I, or a pharmacologically active metabolite thereof, or a pharmaceutically or veterinarily acceptable salt thereof, or a pharmaceutically or veterinarily acceptable solvate of either entity to be administered is 2 to about 600 mg/day, preferably from about 5 to about 400 mg/day, and more preferably about 10 to 300 mg/day. Dosage depends on a number of factors such as age, weight and sex and can be determined by the skilled person. The dosage regimen can also be administered by the skilled person but, for example, a compound of Formula I may be administered once or twice a day, or more regularly such as 3, 4 or 5 times a day (particularly for short-acting compounds).

Preferably, R¹ in a compound of Formula I represents C₁ to C₅ alkyl, more preferably, R¹ represents C₁ to C₄ alkyl, even more preferably linear C₁ to C₄ alkyl. Most preferably, R¹ represents a methyl group.

Preferably, R² in a compound of Formula I is H, fluoro or chloro, more preferably H or chloro. Most preferably, R² is chloro.

Preferably, R³ in a compound of Formula I represents H or C₁ to C₄ alkyl. More preferably, R³ represents H or linear C₁ to C₄ alkyl. Most preferably, R³ represents H.

Preferably, m in a compound of Formula I is an integer from 1 to 6 inclusive, more preferably 2 to 6 inclusive, especially 4 to 6. Most preferably, m is 4.

Particularly preferred compounds of Formula I are: tianeptine (7-[(3-chloro-6,1'-dihydro-6-methyl-dibenzo[c,f][1,2]thiazepin-11-yl)amino]heptanoic acid S,S-dioxide) wherein R¹ is methyl, R² is chloro, R³ is hydrogen, R⁴ is hydrogen, R⁵ is hydrogen, and m is 4 in a compound of Formula I; or the pharmacological active metabolite of tianeptine, referred to as the "MC5 metabolite" (7-[(3-chloro-6,1'-dihydro-6-methyl-dibenzo[c,f][1,2]thiazepin-11-yl)amino]pentanoic acid S,S-dioxide) wherein R¹ is methyl, R² is chloro, R³ is hydrogen, R⁴ is hydrogen, R⁵ is hydrogen, and m is 2 in a compound of Formula I.

In some embodiments, a compound of Formula I is 2-(4-((3-chloro-6-methyl-5,5-dioxido-6,11-dihydrodibenzo[c,f][1,2] thiazepin-11-yl)amino)butoxy)acetic acid, HCI salt (TIOX; Formula 1 where X=O, m=4, R⁶=CO2H; R⁴,R⁵=H; R²=Cl; R¹,R³=Me).

Tianeptine, which has the systematic name 7-[(3-chloro-6,1'-dihydro-6-methyl-dibenzo[c,f][1,2]thiazepin-11-yl)amino]heptanoic acid S,S-dioxide, is a tricyclic anti-depressant of the dibenzothiazepine type. A sodium salt of tianeptine is currently marketed in Europe under the trademark Stablon®. Tianeptine is known to have psychostimulant, antidepressive, analgesic, antitussive, antihistaminic and gastric antisecretory properties. The suggested daily dosage of tianeptine is 37.5 mg, to be given in divided doses three times daily, due to its short duration of action. Tianeptine has a plasma half-life of 2.5 +/- 1.1 h in humans.

As defined herein, the term "C₁ to C₅ alkyl", which R¹ and R³ may each independently represent, may unless otherwise specified, when there is a sufficient number of carbon atoms, be linear or branched, be cyclic, acylic or part cyclic/acyclic. Preferably, the alkyl group is an acyclic alkyl group, more preferably a linear alkyl group. Representative examples of alkyl groups include, but are not limited to, methyl, ethyl, n-propyl, iso-propyl, n-butyl, sec-butyl, isobutyl, tert-butyl, n-pentyl, iso-pentyl, neo-pentyl and hexyl.

The term "halo", when used herein, includes fluoro, chloro, bromo and iodo.

For the avoidance of doubt each R¹, R², and R³ group referred to herein is independent of other R¹, R² and R³ groups, respectively. For example, if R¹ and R³ both represent C₁ to C₅ alkyl then the two individual alkyl substituents are independent of one another, and not necessarily identical (though this possibility is not excluded).

The compounds of Formula I, contain one or more asymmetric carbon atoms and therefore exist in two or more stereoisomeric forms. In a compound of Formula I the aliphatic carbon marked with an asterisk (*) denotes an asymmetric carbon atom and the absolute configuration about that carbon may be (R)- or (S)- as designated according to the Cahn Ingold Prelog system. The present invention includes the individual (R)- and (S)- enantiomeric forms of the compounds of Formula I, in respect of the aliphatic carbon marked with an asterisk (*), and mixtures thereof (e.g. racemates). In accordance with a preferred embodiment, the present invention includes the individual (R)- and (S)-enantiomeric forms of the compounds of Formula I, in respect of the aliphatic carbon marked with an asterisk (*). Accordingly, such individual (R)- and (S)-enantiomeric forms possess optical activity.

As used herein, the individual enantiomeric forms of racemates refer to compositions consisting substantially of a single stereoisomer, i.e. substantially free of the other stereoisomer, that is containing at least 80 %, preferably at least 90 %, more preferably at least 95 %, and even more preferably at least 98 % by weight of such a single stereoisomer. Thus, the term "(R)-enantiomeric form substantially free of the (S)-enantiomeric form" means a compound that comprises at least 80% or more by weight of the (R)-enantiomer (preferably at least 90%, more preferably at least 95 %, and even more preferably at least 98 % by weight of the (R)-enantiomer), and likewise contains 20% or less by weight of the (S)-enantiomer (preferably less than 10 %, more preferably less than 5 %, and even more preferably less than 2 % by weight of the (S)-enantiomer) as a contaminant. By "(S)-enantiomeric form substantially free of the (R)-enantiomeric form" is meant a compound that comprises at least 80% or more by weight of the (S)-enantiomer (preferably at least 90%, more preferably at least 95 %, and even more preferably at least 98 % by weight of the (S)-enantiomer), and likewise contains 20% or less by weight of the (R)-enantiomer (preferably less than 10 %, more preferably less than 5 %, and even more preferably less than 2 % by weight of the (R)-enantiomer) as a contaminant.

As used herein, "optically active" refers to a property whereby a material rotates the plane of plane-polarized light. A compound that is optically active is non-superimposable on its mirror image. As used herein, the property of non-superimposability of an object on its mirror image is called "chirality." The most common structural feature producing chirality is an asymmetric carbon atom; i.e., a carbon atom having four nonequivalent groups attached thereto.

As used herein, "enantiomer" refers to each of the two non-superimposable isomers of a pure compound that is optically active. Single enantiomers are designated according to the Cahn-Ingold-Prelog system, which is a well-known set of priority rules for ranking the four groups attached to an asymmetric carbon. See, e.g., March, Adv Org Chem 4th Ed., (1992), p. 109.

As used herein, "racemate" or "racemic compound" refers to a 50-50 mixture of two enantiomers such that the mixture does not rotate plane-polarized light.

An individual enantiomer of a compound of Formula I, particularly a compound of Formula I in respect of the aliphatic carbon marked with an asterisk (*), may be prepared from the corresponding optically pure intermediate or by resolution, either by HPLC of the racemate using a suitable chiral support or, where appropriate, by fractional crystallisation of the diastereoisomeric salts formed by reaction of the racemate with a suitable optically active acid or base.

It will be appreciated that the compounds of the invention may include one or more further asymmetric carbon atoms, in addition to the aliphatic carbon marked with an asterisk (*) in a compound of Formula I, depending on the identity of each of the substituent groups R¹, R², R³, R⁴ and R⁵. For the avoidance of doubt, all stereoisomers and diastereoisomers of the compounds of Formula I are included within the scope of the invention.

Thus according to a preferred embodiment, the compound of Formula I represents tianeptine as defined hereinbefore, particularly (R)-tianeptine, substantially free of the corresponding (S)-enantiomeric form, with respect to the carbon marked with an asterisk (*) in a compound of Formula I or (S)-tianeptine, substantially free of the corresponding (R)-enantiomeric form, with respect to the carbon marked with an asterisk (*) in a compound of Formula I.

According to a further preferred embodiment, the compound of Formula I represents the MC5 metabolite of tianeptine as defined hereinbefore, particularly the (R)-enantiomeric form, substantially free of the corresponding (S)-enantiomeric form, with respect to the carbon marked with an asterisk (*) in a compound of Formula I or the (S)-enantiomeric form, substantially free of the corresponding (R)-enantiomeric form, with respect to the carbon marked with an asterisk (*) in a compound of Formula I.

To isolate the individual (R)- and (S)-enantiomers of tianeptine, the racemate must be resolved. This resolution can be achieved by converting racemic tianeptine into a pair of diastereomers, for example by covalently bonding to an optically active moiety or by salt formation with an optically active base or acid. Either method provides a molecule with a second chiral center, thus generating a pair of diastereomers. The diastereomeric pair can then be separated by conventional methods, such as crystallization or chromatography.

Racemic tianeptine can also be separated into enantiomers without diastereomer formation, for example, by differential absorption on a chiral stationary phase of a chromatography (e.g., HPLC) column. Preparative HPLC columns suitable for diastereomer separation are commercially available with a variety of packing materials to suit a broad range of separation applications. Stationary phases suitable for resolving tianeptine include: (i) macrocyclic glycopeptides, such as silica-bonded vancomycin which contains 18 chiral centers surrounding three pockets or cavities; (ii) chiral α₁-acid glycoprotein; (iii) human serum albumin; and (iv) cellobiohydrolase (CBH).

The compounds of Formula I, such as tianeptine and the MC5 metabolite, may be prepared by known synthetic procedures, for example as described in: French patent 2,104,728; GB patent application 1,269,551; U.S. patents Nos. 4,766,114, 3,758,528 and 3,821,249, all of Malen et al.; and U.S. patent No. 6,441,165 of Blanchard et al. Compounds of Formula I where X=O or S can be prepared according to methods described in WO2012143703.

The pharmaceutically or veterinarily acceptable salts of the compounds of Formula I are, for example, non-toxic acid addition salts formed with inorganic acids or organic acids or base addition salts. Suitable inorganic acids include hydrochloric, hydrobromic, hydroiodic, nitric, carbonic, sulfuric and phosphoric acid. Suitable organic acids include aliphatic, cycloaliphatic, aromatic, araliphatic, heterocyclic, carboxylic and sulfonic classes of organic acids, such as formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, salicylic, 4-hydroxybenzoic, phenylacetic, mandelic, embonic (pamoic), methanesulfonic, ethanesulfonic, benzenesulfonic, pantothenic, 2-hydroxyethanesulfonic, toluenesulfonic, sulfanilic, cyclohexylaminosulfonic, stearic, algenic, beta-hydroxybutyric, galactaric and galacturonic acid.

Suitable pharmaceutically acceptable base addition salts of the compounds of Formula I include metallic salts made from calcium, magnesium, potassium, sodium and zinc, or organic salts made from N,N'-dibenzylethylenediamine, chloroprocaine, choline, diethanolamine, ethylenediamine, meglumine (N-methylglucamine), arginine and procaine.

The pharmaceutically acceptable acid addition salts of the compounds of Formula I may be prepared in a conventional manner. For example, a solution of the free base is treated with the appropriate acid, either neat or in a suitable solvent, and the resulting salt isolated either by filtration or by evaporation under vacuum of the reaction solvent. Pharmaceutically acceptable base addition salts can be obtained in an analogous manner by treating a solution of a compound of Formula I with the appropriate base. Both types of salt may be formed or interconverted using ion-exchange resin techniques. For a review on suitable pharmaceutical salts see Berge et. al., J. Pharm., Sci., 66, 1-19, 1977. A highly preferred salt is the sodium salt.

The pharmaceutically or veterinarily acceptable solvates of the compounds of Formula I include the hydrates thereof.

Also included in the invention are radiolabelled and isotopically labeled derivatives of the compounds of Formula I which are suitable for biological studies. Examples of such derivatives include, but are not limited to, ²H, ³H, ¹¹C, ¹³C, ¹⁴C, ¹⁵N, ¹⁷O, ¹⁸O, ¹⁸F, ³⁵S and ³⁶Cl.

Certain compounds of Formula I may exist in multiple crystalline or amorphous forms. All physical forms and polymorphs are included within the scope of the invention.

It will be appreciated by those skilled in the art that certain derivatives of compounds of Formula I may not possess pharmacological activity as such, but may, in certain instances, be administered orally or parenterally and thereafter metabolized in the body to form compounds of Formula I which are pharmacologically active. Such derivatives may therefore be described as "prodrugs". Further, certain compounds of Formula I may act as prodrugs of other compounds of Formula I. All prodrugs, of compounds of Formula I are included within the scope of the invention.

Additionally, the compound of Formula I may be metabolized in the body of the subject to form an active metabolite. The administration of such metabolites to treat movement disorders is also contemplated within the scope of the invention. Tianeptine is metabolised to 7-[(3-chloro-6,1'-dihydro-6-methyl-dibenzo[c,f][1,2]thiazepin-11-yl)amino]pentanoic acid S,S-dioxide, an active metabolite known as the "MC5 metabolite". Thus, according to a preferred aspect the present the present invention extends to the use of tianeptine and the MC5 metabolite, or a pharmaceutically or veterinarily acceptable solvate of either entity or a pharmaceutical or veterinary composition containing any of the foregoing for the treatment of movement disorders in mammal, such as an animal or human, especially a human.

### Medical Use

The compounds of Formula I are useful because they possess pharmacological activity for the treatment of CDKL5 disorder in a subject (i.e. mammals, especially humans). They are therefore indicated as pharmaceuticals, as well as for use as animal medicaments for reducing or inhibiting CDKL5 disorder in animals and humans.

The terms "reducing" or "inhibiting" as used herein refers to a reduction in CDKL5 disorder in a subject in the presence of a compound of Formula I, preferably tianeptine, as compared with the level of CDKL5 disorder in the absence of such a compound.

By the term "subject" as referred to herein we mean "a mammal" which includes animals and humans, especially humans. The term "mammal" therefore may also include domestic and common laboratory mammals such as non-human primates, horses, pigs, goats, sheep, dogs, cats, rabbits, mice, rats, and the like. The most preferred mammal is a human subject.

The methods and compositions of the present invention are directed toward subjects having CDKL5 disorder. Thus the invention provides a method of treating, such as reducing or inhibiting, CDKL5 disorder in a subject (i.e. mammal) in need of such treatment comprising administering to the subject a therapeutic effective amount of a compound of Formula I as defined herein, or a pharmaceutically or veterinarily acceptable salt thereof, or a pharmaceutically or veterinarily acceptable solvate of either entity or a pharmaceutical or veterinary composition containing any of the foregoing. Preferably, the compound of Formula I is tianeptine or the MC5 metabolite.

Thus the invention also provides the use of a compound of Formula I, or a pharmaceutically or veterinarily acceptable salt thereof, or a pharmaceutically or veterinarily acceptable solvate of either entity or a pharmaceutical or veterinary composition containing any of the foregoing for the treatment of CDKL5 disorder in a subject (i.e. mammal). Preferably, the compound of Formula I is tianeptine or the MC5 metabolite, or a compound of Formula I wherein R¹ and R³ each represent methyl, R² represents chloro, R⁴ and R⁵ each independently represent H, R⁶ represents -C(O)OH, X represents O and m is 4.

### Pharmaceutical Preparation

The compounds of Formula I will normally be administered orally or by any parenteral route in the form of pharmaceutical preparations comprising the active ingredient, optionally in the form of a non-toxic organic, or inorganic, acid, or base, addition salt, in a pharmaceutically acceptable dosage form. Depending upon the disorder and patient to be treated, as well as the route of administration, the compositions may be administered at varying doses.

One skilled in the art can readily determine an effective amount of a compound of Formula I to be administered, by taking into account factors such as the size, weight, age and sex of the subject, the extent of disease penetration or persistence and severity of symptoms, and the route of administration. Generally, an effective amount of a compound of Formula I, such as tianeptine, administered to a subject is from about 2 to about 600 mg/day, preferably from about 5 to about 400 mg/day, and more preferably about 10 to 300 mg/day. Higher or lower doses are also contemplated.

The compound of Formula I can be administered to a subject by any route, for example by enteral (e.g., oral, rectal, intranasal, etc.) and parenteral administration. Parenteral administration includes, for example, intravenous, intramuscular, intraarterial, intraperitoneal (ip), intravaginal, intravesical (e.g., into the bladder), intradermal, topical or subcutaneous administration. Also contemplated within the scope of the invention is the instillation of the compound of Formula I into the body of the subject, for example in a controlled release formulation, with systemic or local release of the compound to occur over time or at a later time. Preferably, the compound of Formula I, e.g. tianeptine, is localized in a depot for controlled release to the circulation or to a local site such as the gastrointestinal tract.

A compound of Formula I, e.g. tianeptine, can be administered together with a pharmaceutically or veterinarily acceptable carrier. Pharmaceutical formulations can comprise from 0.1 to 99.99 weight percent of a compound of Formula I, e.g. tianeptine. The pharmaceutical compositions can be formulated according to standard practices in the field of pharmaceutical preparations. See Alphonso Gennaro, ed., Remington's Pharmaceutical Sciences. 18th Ed., (1990) Mack Publishing Co., Easton, Pa. Suitable dosage forms can comprise, for example, tablets, capsules, solutions, parenteral solutions, troches, suppositories, or suspensions.

By "pharmaceutically acceptable carrier" is meant any diluent or excipient that is compatible with the other ingredients of the composition, and which is not deleterious to the recipient. The pharmaceutically acceptable carrier can be selected on the basis of the desired route of administration, in accordance with standard pharmaceutical practices.

Pharmaceutical compositions for parenteral administration can take the form of an aqueous or nonaqueous solution, dispersion, suspension or emulsion. In preparing pharmaceutical compositions for parenteral administration, a compound of Formula I, e.g. tianeptine, can be mixed with a suitable pharmaceutically acceptable carrier such as water, oil (particularly a vegetable oil), ethanol, saline solutions (e.g., normal saline), aqueous dextrose (glucose) and related sugar solutions, glycerol, or glycols such as propylene glycol or polyethylene glycol. Pharmaceutical compositions for parenteral administration preferably contain a water-soluble salt of the compound of Formula I, e.g. tianeptine. Stabilizing agents, antioxidizing agents and preservatives can also be added to the pharmaceutical compositions for parenteral administration. Suitable antioxidizing agents include sulfite, ascorbic acid, citric acid and its salts, and sodium EDTA. Suitable preservatives include benzalkonium chloride, methyl- or propyl-paraben, and chlorbutanol.

In preparing pharmaceutical compositions for oral administration, the compound of Formula I, e.g. tianeptine, can be combined with one or more solid or liquid inactive ingredients to form tablets, capsules, pills, powders, granules or other suitable oral dosage forms. For example, the compound of Formula I, e.g. tianeptine, can be combined with at least one pharmaceutically acceptable carrier such as a solvent, filler, binder, humectant, disintegrating agent, solution retarder, absorption accelerator, wetting agent absorbent or lubricating agent. In one embodiment, the compound of Formula I, e.g. tianeptine, is combined with carboxymethylcellulose calcium, magnesium stearate, mannitol and starch, and is formed into tablets by conventional tableting methods.

In one embodiment, controlled-release pharmaceutical compositions comprise the compound of Formula I, e.g. tianeptine, and a controlled-release component. Preferably, a controlled-release pharmaceutical composition is capable of releasing the compound of Formula I, e.g. tianeptine, into a subject at a desired rate, so as to maintain a substantially constant pharmacological activity for a given period of time. As used herein, a "controlled-release component" is a compound such as a polymer, polymer matrix, gel, permeable membrane, liposome and/or microsphere that induces the controlled-release of the compound of Formula I, e.g. tianeptine, into the subject upon exposure to a certain physiological compound or condition. For example, the controlled-release component can be biodegradable, activated by exposure to a certain pH or temperature, by exposure to an aqueous environment, or by exposure to enzymes. An example of a controlled-release component which is activated by exposure to a certain temperature is a sol-gel. In this embodiment, tianeptine is incorporated into a sol-gel matrix that is a solid at room temperature. This sol-gel matrix is implanted into a subject having a body temperature high enough to induce gel formation of the sol-gel matrix, thereby releasing the active ingredient into the subject. Suitable controlled release formulations are described in, for example, U.S. Pat. No. 5,674,533 (liquid dosage forms), U.S. Pat. No. 5,591,767 (liquid reservoir transdermal patch), U.S. Pat. No. 5,120,548 (device comprising swellable polymers), U.S. Pat. No. 5,073,543 (ganglioside-liposome vehicle), U.S. Pat. No. 5,639,476 (stable solid formulation coated with a hydrophobic acrylic polymer) and U.S. Pat. 5,888,542 (matrix tablet allowing the prolonged release of the sodium salt of tianeptine after administration by the oral route. Biodegradable microparticles can also be used to formulate suitable controlled-release pharmaceutical compositions, for example as described in U.S. Pat. 5,354,566 and 5,733,566.

Generally, in humans oral or intravenous administration of the compounds of Formula I in the form of a pharmaceutical formulation is the preferred route.

Thus, the invention also provides a pharmaceutical composition for use in the treatment of CDKL5 disorder in a human the composition comprising a compound of Formula I as defined herein or a pharmaceutically acceptable salt thereof, or a pharmaceutically acceptable solvate of either entity, in admixture with a pharmaceutically acceptable adjuvant, diluent or carrier. Suitably, the invention also extends to a method of treating, such as inhibiting or reducing, CDKL5 disorder in a human by administering such a pharmaceutical composition to a human. Suitably, the invention extends to the use of such a pharmaceutical composition for treating CDKL5 disorder in a human.

Thus, in accordance with a further aspect the present invention provides the use of a compound of Formula I as defined herein or a pharmaceutically acceptable salt thereof, or a pharmaceutically acceptable solvate of either entity, in the manufacture of a medicament for use in the treatment of CDKL5 disorder in a human.

According to a further aspect of the invention there is provided a veterinary composition for use in the treatment of CDKL5 disorder in an animal comprising a compound of Formula I, or a veterinarily acceptable salt thereof, or a veterinarily acceptable solvate of either entity, in admixture with a veterinarily acceptable adjuvant, diluent or carrier. Suitably, the invention also extends to a method of treating, such as inhibiting or reducing, CDKL5 disorder in an animal by administering such a veterinary composition to an animal. Suitably, the invention extends to the use of such a veterinary composition for treating or preventing CDKL5 disorder in an animal.

Thus according to a further aspect, the present invention provides a pharmaceutical composition for use in the treatment of CDKL5 disorder in a human the pharmaceutical composition of a compound of Formula I as defined herein or a pharmaceutically acceptable salt thereof, or a pharmaceutically acceptable solvate of either entity, in admixture with a pharmaceutically acceptable adjuvant, diluent or carrier. Preferably, such a pharmaceutical composition is for oral administration.

For oral and parenteral administration to human patients, the daily dosage level of the compounds of Formula I or salts or solvates thereof will usually be from 2 to about 600 mg/day, preferably from about 5 to about 400 mg/day, and more preferably about 10 to 300 mg/day.

Thus, for example, tablets or capsules of the compounds of Formula I or salts or solvates thereof may contain from 2.5 mg to 250 mg of active compound for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention.

The invention will now be exemplified by the following non-limiting examples.

### Examples

### Example 1: Tablet Formulation

In general a tablet formulation could typically contain between about 2.5 mg and 250 mg of a compound of Formula I (or a salt thereof) whilst tablet fill weights may range from 50 mg to 1000 mg. An example formulation for a 250 mg tablet is illustrated:

| Ingredient | %w/w |
|---|---|
| Tianeptine Na salt | 10 |
| Lactose | 65 |
| Starch | 21 |
| Croscarmellose Sodium | 3 |
| Magnesium Stearate | 2 |

### Example 2: CDKL5 gene silencing experiment

### Methods

*Neuronal cultures* - Primary hippocampal cultures were prepared from hippocampi of embryonic day 17 (E17) CD1 mouse embryos, considering the day of the vaginal plug as E0. Briefly, neurons were dissociated in DMEM with 10% horse serum (HS, Life Technologies) by gentle trituration and seeded in Neurobasal containing 2% B27 supplement and 2 mM glutamine on poly-L-lysine coated dishes or coverslips and incubated at 37°C with 5% CO₂. The density of neurons is 3.7x10⁴/cm² for western blots and immunostainings.

*CDKL5 knock-down* - CDKL5 expression was silenced by the addition of lentiviral particles to the neurons before plating. Lentiviral vectors contain the shRNA-coding sequence cloned into the Hpal and Xhol sites of pLentiLox 3.7 (pLL 3.7). The target sequences of the shRNAs are as follows: shCDKL5#1: CTATGGAGTTGTACTTAA; shCDKL5#2: GCAGAGTCGGCACAGCTAT; a shRNA against LacZ was used as control. The constructs express GFP from an independent promoter allowing visualizing infected neurons.

*Western blotting* - AMPA-R expression was analyzed at 18-21 days in vitro (DIV). Neurons are harvested in Laemmli buffer and an amount of extract corresponding to approximately 10 µg is separated by SDS-PAGE (8%). The following antibodies are used to confirm silencing of CDKL5 and analysing GluR1/GluR2 expression and GluR2-Ser880 phosphorylation: CDKL5 1:1000 (Sigma Aldrich HPA002847), GluR1 1:1000 (Abcam, ab86141), GluR2 1:1000 (Millipore, MAB397), GluR2 phospho-Ser880 1:500 (Biorbyt orb256572), and Tubulin β3 1:10000 (Biolegend, 811801).

*Immunofluorescence* - Neurons were fixed at DIV18 with 4% paraformaldehyde/PBS for 15 min and incubated with blocking solution (5% Horse serum in PBS) in non-permeabilizing conditions for 1 h at room temperature and then with primary antibodies (overnight at 4°C) in blocking solution. Primary antibodies against extracellular epitopes of GluR2 were used for the quantification of cell-surface expressed GluR2. Subsequently anti-MAP2 was added in a permeabilizing solution (blocking solution w. 0.2% Triton X-100) After 3 rinses, cells were incubated with secondary antibodies (Molecular Probes) for 1 h at room temperature. Coverslips were mounted for microscopy using ProLong Gold Antifade reagent (Molecular Probes, P36930). Antibodies were used in the following concentrations: GluR2 1:100 (Millipore, MAB397), MAP2 1:1000 (Abcam, ab11267), GFP 1:1000 (Millipore, AB16901). Microscopic analysis was performed on an Olympus BX51 Fluorescence microscope equipped with Retiga R1 (Qlmaging) CCD camera. Quantification of surface-expressed GluR2 was performed with Image J software on segments of GFP- and MAP2-posititve neurites.

*Pharmacologic treatment* - Tianeptine and 2-(4-((3-chloro-6-methyl-5,5-dioxido-6,11-dihydrodibenzo[c,f][1,2]thiazepin-11-yl)amino)butoxy)acetic acid [HCI salt] were added to the neuronal cultures (final concentration 10 µM) starting at DIV11 and replenished every second day (DIV13, 15, and 17) till DIV18.embryonic day 17 (E17) CD1 mouse embryos, considering the day of the vaginal plug as E0. Briefly, neurons were dissociated in DMEM with 10% horse serum (HS, Life Technologies) by gentle trituration and seeded in Neurobasal containing 2% B27 supplement and 2 mM glutamine on poly-L-lysine coated dishes or coverslips and incubated at 37°C with 5% CO₂. The density of neurons is 3.7x10⁴/cm² for western blots and immunostainings.

### Results

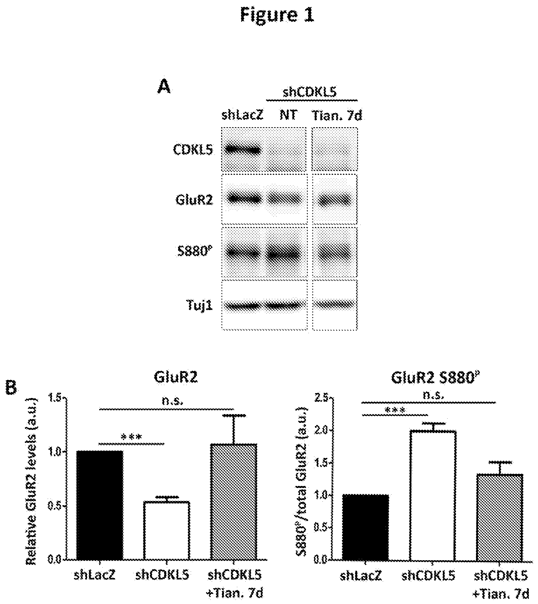

From Figure 1, it can be seen that administration of tianeptine sulphate (10uM) on alternate days from DIV11 to DIV18 (DIV11-13-15-17) reversed the effect of CDKL5 silencing on expression of the AMPA receptor subunit GluR2 and on phosphorylated GluR2 (phosphorylation at serine-880) as determined by Western blotting.

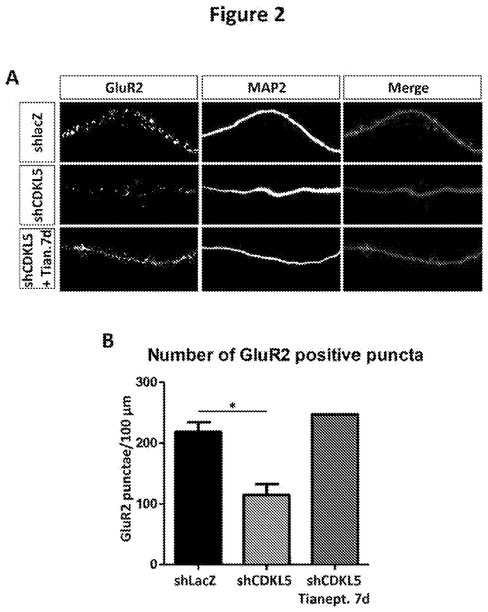

From Figure 2, it can be seen that administration of tianeptine sulphate (10uM) on alternate days from DIV11 to DIV18 (DIV11-13-15-17) reversed the effect of CDKL5 silencing on expression of the AMPA receptor subunit GluR2 in terms of number of puncta as determined by immunofluorescence.

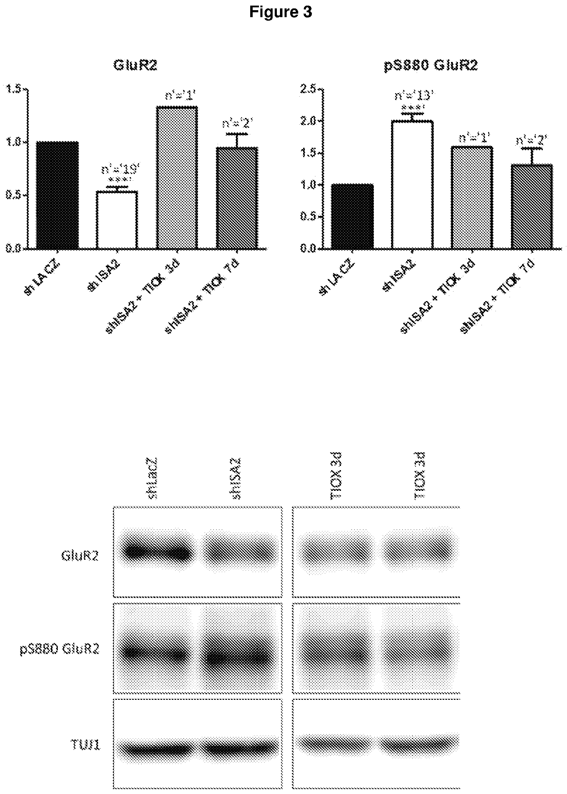

From Figure 3, it can be seen that administration of 2-(4-((3-chloro-6-methyl-5,5-dioxido-6,11-dihydrodibenzo[c,f][1,2] thiazepin-11-yl)amino)butoxy)acetic acid [HCI salt] (TIOX) (10 µM) on alternate days from DIV15-18 (DIV15-17) or DIV11 to DIV18 (DIV11-13-15-17) reversed the effect of CDKL5 silencing on expression of the AMPA receptor subunit GluR2 and on phosphorylated GluR2 (phosphorylation at serine-880) as determined by Western blotting. GluR2 is also a post-synaptic marker and CDKL5-modulated deficits in this protein are shown to be addressed using compounds of the invention.

In summary, tianeptine and TIOX both reversed the effects of CDKL5 gene silencing and therefore it is expected that these compounds (and the analogues within the scope of the present invention) would be useful in the therapy of CDKL5 disorder.

### Example 3: Improvement of motor coordination in animal model of CDKL5 disorder

The generation of *Cdkl5* knockout mice and rotarod assessment of motor coordination could be carried out following the method described in Wang, I.-T.J et al. (2012) PNAS 109: 21516-21521, as follows.

Briefly, motor performance is evaluated in separate cohorts of *Cdkl5* knockout mice given tianeptine beginning at 6 weeks of age, to evaluate effects in symptomatic mice, and at 3 weeks of age, to assess effects prior to the onset of major motor impairments. In the first group tested, tianeptine is administered to 6 week old *Cdkl5* knockout and wild-type (WT) mice (n=7-8/group) for a period of 4 weeks. Motor performance is assessed using a rotarod one week before (baseline) and once per week during treatment; the latter trials occur 18 hours after the preceding tianeptine injection. Results would be expected as follows: vehicle-treated *Cdkl5* knockout mice exhibit substantial rotarod impairment by 6 weeks post-natal. After 4 weeks of treatment and testing, scores for *Cdkl5* knockout mice treated with tianeptine are higher than scores for *Cdkl5* knockout mice given vehicle but are below WT values. Thus, 4 weeks of tianeptine treatment, initiated after the onset of motor deficits, improves scores in a test of motor coordination and balance in *Cdkl5* knockout mice. Similarly, in *Cdkl5* knockout mice treated before the onset of motor deficits, at 3 weeks of age, tianeptine is able to increase the time spent on the rotarod before falling relative to untreated mice.

## Claims

1. A compound of Formula I, or a pharmaceutically or veterinarily acceptable salt thereof, or a pharmaceutically or veterinarily acceptable solvate of either entity, for use in the treatment of CDKL5 disorder in a mammal, wherein a compound of Formula I comprises: 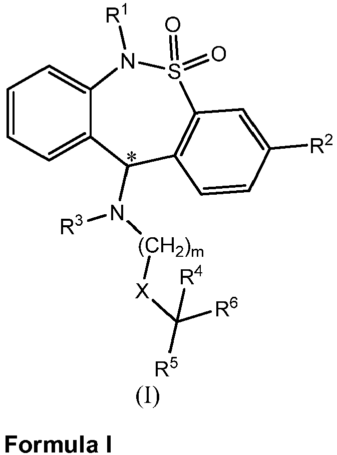 or a pharmaceutically or veterinarily acceptable salt thereof, or a pharmaceutically or veterinarily acceptable solvate of either entity,
wherein:
R¹ and R³ each independently represent, at each occurrence when used herein,
H or C₁ to C₅ alkyl;
R² represents halo;
R⁴ and R⁵ each independently represent H;
R⁶ represents -C(O)OR⁹;
X represents CH₂, O or S;
R⁹ represents H or C₁ to C₅ alkyl;
and,
m is an integer from 1 to 6 inclusive.

2. A compound of Formula I for use in the treatment of CDKL5 disorder in a mammal as claimed in claim 1, wherein R¹ in a compound of Formula I represents a C₁ to C₄ alkyl group.

3. A compound of Formula I for use in the treatment of CDKL5 disorder in a mammal as claimed in claim 1 or 2, wherein R² in a compound of Formula I represents halo.

4. A compound of Formula I for use in the treatment of CDKL5 disorder in a mammal as claimed in any one of the preceding claims, wherein R³ in a compound of Formula I represents H.

5. A compound of Formula I for use in the treatment of CDKL5 disorder in a mammal as claimed in any one of the preceding claims, wherein m in a compound of Formula I is an integer from 2 to 6 inclusive.

6. A compound of Formula I for use in the treatment of CDKL5 disorder in a mammal as claimed in claim 1, wherein the compound of Formula I is tianeptine such that in a compound of Formula I R¹ is methyl, R² is chloro, R³ is hydrogen, R⁴ is hydrogen, R⁵ is hydrogen, and m is 4.

7. A compound of Formula I for use in the treatment of CDKL5 disorder in a mammal as claimed in claim 1, wherein in a compound of Formula I is the MC5 metabolite of tianeptine such that in a compound of Formula I R¹ is methyl, R² is chloro, R³ is hydrogen, R⁴ is hydrogen, R⁵ is hydrogen, and m is 2.

8. A compound of Formula I for use in the treatment of CDKL5 disorder in a mammal as claimed in any one of the preceding claims, wherein the compound of Formula I is in the (R)-enantiomeric form in respect of the aliphatic carbon marked with an asterisk (*) and substantially free of the (S)-enantiomeric form in respect of the aliphatic carbon marked with an asterisk (*).

9. A compound of Formula I for use in the treatment of CDKL5 disorder in a mammal as claimed in any one of claims 1 to 7, wherein the compound of Formula I is in the (S)-enantiomeric form in respect of the aliphatic carbon marked with an asterisk (*) and substantially free of the (R)-enantiomeric form in respect of the aliphatic carbon marked with an asterisk (*).

10. A pharmaceutical composition for use in the treatment of CDKL5 disorder in a human the composition comprising a therapeutically effective amount of a compound of Formula I as defined in any one of the preceding claims or a pharmaceutically acceptable salt thereof, or a pharmaceutically acceptable solvate of either entity, in admixture with a pharmaceutically acceptable adjuvant, diluent or carrier.

11. A compound of Formula I as claimed in any one of claims 1 to 9 for use in the treatment of CDKL5 disorder in a mammal, wherein the mammal is a human.

12. A veterinary composition for use in the treatment of CDKL5 disorder in an animal comprising a compound of Formula I as defined in any one of claims 1 to 9, or a veterinarily acceptable salt thereof, or a veterinarily acceptable solvate of either entity, in admixture with a veterinarily acceptable adjuvant, diluent or carrier.

## Patentansprüche

1. Verbindung der Formel I oder ein pharmazeutisch oder veterinärmedizinisch annehmbares Salz davon oder ein pharmazeutisch oder veterinärmedizinisch annehmbares Solvat einer der beiden Einheiten zur Verwendung bei der Behandlung der CDKL5-Störung bei einem Säugetier, wobei eine Verbindung der Formel I Folgendes umfasst: 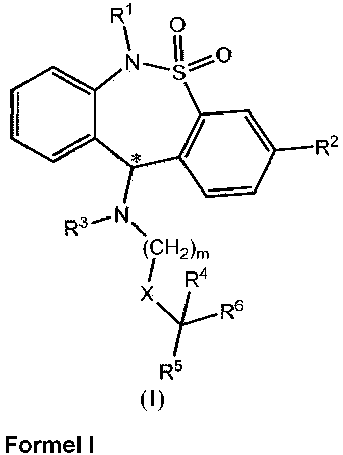 oder ein pharmazeutisch oder veterinärmedizinisch annehmbares Salz davon, oder ein pharmazeutisch oder veterinärmedizinisch annehmbares Solvat einer der beiden Einheiten, worin:
R¹ und R³ jeweils unabhängig voneinander bei jedem Auftreten, wenn sie hierin verwendet werden, für H oder C₁ to C₅-Alkyl stehen;
R² für Halogen steht;
R⁴ und R⁵ jeweils unabhängig voneinander für H stehen;
R⁶ für -C(O)OR⁹ steht;
X für CH₂, O oder S steht;
R⁹ für H oder C₁- bis C₅-Alkyl steht; und,
m eine ganze Zahl von 1 bis einschließlich 6 ist.

2. Verbindung der Formel I zur Verwendung bei der Behandlung der CDKL5-Störung bei einem Säugetier nach Anspruch 1, wobei R¹ in einer Verbindung der Formel I für eine C₁-C₄-Alkylgruppe steht.

3. Verbindung der Formel I zur Verwendung bei der Behandlung der CDKL5-Störung bei einem Säugetier nach Anspruch 1 oder 2, wobei R² in einer Verbindung der Formel I für Halogen steht.

4. Verbindung der Formel I zur Verwendung bei der Behandlung der CDKL5-Störung bei einem Säugetier nach einem der vorhergehenden Ansprüche, wobei R³ in einer Verbindung der Formel I für H steht.

5. Verbindung der Formel I zur Verwendung bei der Behandlung der CDKL5-Störung bei einem Säugetier nach einem der vorhergehenden Ansprüche, wobei m in einer Verbindung der Formel I eine ganze Zahl von 2 bis einschließlich 6 ist.

6. Verbindung der Formel I zur Verwendung bei der Behandlung der CDKL5-Störung bei einem Säugetier nach Anspruch 1, wobei die Verbindung der Formel I Tianeptin ist, so dass in einer Verbindung der Formel I R¹ Methyl ist, R² Chlor ist, R³ Wasserstoff ist, R⁴ Wasserstoff ist, R⁵ Wasserstoff ist und m 4 ist.

7. Verbindung der Formel I zur Verwendung bei der Behandlung der CDKL5-Störung bei einem Säugetier nach Anspruch 1, wobei in einer Verbindung der Formel I der MC5-Metabolit von Tianeptin ist, so dass in einer Verbindung der Formel I R¹ Methyl ist, R² Chlor ist, R³ Wasserstoff ist, R⁴ Wasserstoff ist, R⁵ Wasserstoff ist und m 2 ist.

8. Verbindung der Formel I zur Verwendung bei der Behandlung der CDKL5-Störung bei einem Säugetier nach einem der vorhergehenden Ansprüche, wobei die Verbindung der Formel I in der (R)-enantiomeren Form in Bezug auf den mit einem Sternchen (*) markierten aliphatischen Kohlenstoff und im Wesentlichen frei von der (S)-enantiomeren Form in Bezug auf den mit einem Sternchen (*) markierten aliphatischen Kohlenstoff ist.

9. Verbindung der Formel I zur Verwendung bei der Behandlung der CDKL5-Störung bei einem Säugetier nach einem der Ansprüche 1 bis 7, wobei die Verbindung der Formel I in der (S)-enantiomeren Form in Bezug auf den mit einem Sternchen (*) markierten aliphatischen Kohlenstoff und im Wesentlichen frei von der (R)-enantiomeren Form in Bezug auf den mit einem Sternchen (*) markierten aliphatischen Kohlenstoff ist.

10. Pharmazeutische Zusammensetzung zur Verwendung bei der Behandlung der CDKL5-Störung bei einem Menschen, wobei die Zusammensetzung eine therapeutisch wirksame Menge einer Verbindung der Formel I, wie in einem der vorstehenden Ansprüche definiert, oder eines pharmazeutisch annehmbaren Salzes davon oder eines pharmazeutisch annehmbaren Solvats einer der beiden Einheiten im Gemisch mit einem pharmazeutisch annehmbaren Adjuvans, Verdünnungsmittel oder Träger umfasst.

11. Verbindung der Formel I nach einem der Ansprüche 1 bis 9 zur Verwendung bei der Behandlung der CDKL5-Störung bei einem Säugetier, wobei das Säugetier ein Mensch ist.

12. Veterinärmedizinische Zusammensetzung zur Verwendung bei der Behandlung der CDKL5-Störung bei einem Tier, umfassend eine Verbindung der Formel I, wie in einem der Ansprüche 1 bis 9 definiert, oder ein veterinärmedizinisch annehmbares Salz davon oder ein veterinärmedizinisch annehmbares Solvat einer der beiden Einheiten im Gemisch mit einem veterinärmedizinisch annehmbaren Adjuvans, Verdünnungsmittel oder Träger.

## Revendications

1. Composé de formule I, ou un sel pharmaceutiquement ou vétérinairement acceptable de celui-ci, ou un solvate pharmaceutiquement ou vétérinairement acceptable de l'une ou l'autre entité, destiné à être utilisé dans le traitement du syndrome CDKL5 chez un mammifère, dans lequel un composé de formule I comprend : 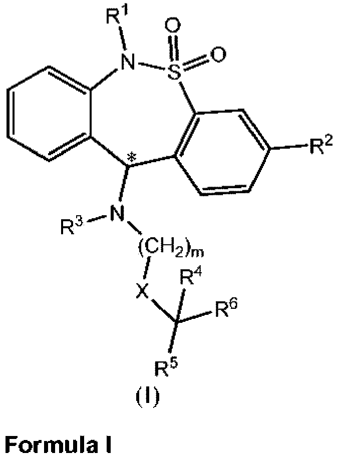 ou un sel pharmaceutiquement ou vétérinairement acceptable de celui-ci, ou un solvate pharmaceutiquement ou vétérinairement acceptable de l'une ou l'autre entité, dans lequel :
R¹ et R³ représentent chacun indépendamment, à chaque occurrence lorsqu'ils sont utilisés ici,
H ou un alkyle en C₁ à C₅ ;
R² représente un halo ;
R⁴ et R⁵ représentent chacun indépendamment H ;
R⁶ représente -C(O)OR⁹ ;
X représente CH₂, O ou S ;
R⁹ représente H ou un alkyle en C₁ à C₅; et,
m est un entier de 1 à 6 inclus.

2. Composé de formule I destiné à être utilisé dans le traitement du syndrome CDKL5 chez un mammifère selon la revendication 1, dans lequel R¹ dans un composé de formule I représente un groupe alkyle en C₁ à C₄.

3. Composé de formule I destiné à être utilisé dans le traitement du syndrome CDKL5 chez un mammifère selon la revendication 1 ou 2, dans lequel R² dans un composé de formule I représente un halo.

4. Composé de formule I destiné à être utilisé dans le traitement du syndrome CDKL5 chez un mammifère selon l'une quelconque des revendications précédentes, dans lequel R³ dans un composé de formule I représente H.

5. Composé de formule I destiné à être utilisé dans le traitement du syndrome CDKL5 chez un mammifère selon l'une quelconque des revendications précédentes, dans lequel m dans un composé de formule I est un entier de 2 à 6 inclus.

6. Composé de formule I destiné à être utilisé dans le traitement du syndrome CDKL5 chez un mammifère selon la revendication 1, dans lequel le composé de formule I est la tianeptine de telle sorte que dans un composé de formule I R¹ est méthyle, R² est chloro, R³ est hydrogène, R⁴ est hydrogène, R⁵ est hydrogène et m est 4.

7. Composé de formule I destiné à être utilisé dans le traitement du syndrome CDKL5 chez un mammifère selon la revendication 1, dans lequel dans un composé de formule I se trouve le métabolite MC5 de la tianeptine de telle sorte que dans un composé de formule I R¹ est méthyle, R² est chloro, R³ est hydrogène, R⁴ est hydrogène, R⁵ est hydrogène et m est 2.

8. Composé de formule I destiné à être utilisé dans le traitement du syndrome CDKL5 chez un mammifère selon l'une quelconque des revendications précédentes, dans lequel le composé de formule I est sous la forme (R)-énantiomère par rapport au carbone aliphatique marqué d'un astérisque (*) et sensiblement exempt de la forme (S)-énantiomère par rapport au carbone aliphatique marqué d'un astérisque (*).

9. Composé de formule I destiné à être utilisé dans le traitement du syndrome CDKL5 chez un mammifère selon l'une quelconque des revendications 1 à 7, dans lequel le composé de formule I est sous la forme (S)-énantiomère par rapport à l'aliphatique carbone marqué d'un astérisque (*) et sensiblement exempt de la forme (R) -énantiomère par rapport au carbone aliphatique marqué d'un astérisque (*).

10. Composition pharmaceutique destinée à être utilisée dans le traitement du syndrome CDKL5 chez un être humain, la composition comprenant une quantité thérapeutiquement efficace d'un composé de formule I tel que défini selon l'une quelconque des revendications précédentes ou un sel pharmaceutiquement acceptable de celui-ci, ou un solvate pharmaceutiquement acceptable de l'une ou l'autre entité, en mélange avec un adjuvant, diluant ou support pharmaceutiquement acceptable.

11. Composé de formule I selon l'une quelconque des revendications 1 à 9 destiné à être utilisé dans le traitement du syndrome CDKL5 chez un mammifère, dans lequel le mammifère est un être humain.

12. Composition vétérinaire destinée à être utilisée dans le traitement du syndrome CDKL5 chez un animal comprenant un composé de formule I tel que défini selon l'une quelconque des revendications 1 à 9, ou un sel vétérinairement acceptable de celui-ci, ou un solvate vétérinairement acceptable de l'une ou l'autre entité, en mélange avec un adjuvant, diluant ou support vétérinairement acceptable.
